# EUROPEAN PATENT APPLICATION

(11) **EP 1 559 363 A2**
(43) Date of publication of application: **03.08.2005**
(21) Application number: 05250533.6
(22) Date of filing: 01.02.2005
(51) Int. Cl.: A61B 1/04, A61B 5/00, A61B 5/055, A61B 8/12

(54) **Combined intra-rectal optical-MR and Intrarectal optical-US device for prostate-, cevix-, rectum imaging dianostics**

(30) Priority: 02.02.2004 US 541020 P; 27.01.2005 US 44239 P
(71) Applicant: Siemens Aktiengesellschaft, 80333 München (DE)
(72) Inventor: Von Rueckmann, Bogdan, 81373 München (DE); Gareus, Ralph, 91031 Forchheim (DE); Hengerer, Arne, 91054 Erlangen (DE); Weissleder, Ralph, Peabody, MA 01960 (US); Durr, Wilhelm, 91056 Erlangen (DE)
(74) Representative: Vigars, Christopher Ian

(57) **Abstract**

We present, in exemplary embodiments of the present invention, a system combining optical imaging technologies with anatomical imaging technologies (e.g., MR, ultrasound). The system can be used for a variety of applications, including, but not limited to, (1) cancer diagnosis and staging; (2) image guidance; and (3) radiation therapy planning. Image guidance may include guiding a biopsy. For example, a prostatectomy potentially has severe side effects, such as impotence and incontinence. Thus, a histologically-confirmed diagnosis, such as one provided from a biopsy, may prevent unnecessary prostatectomy. Image guidance may also include guiding minimal invasive therapy, such as brachytherapy focused ultrasound. The present invention may be used to plan radiation therapy, for example, by detecting, and thus sparing, healthy tissue from radiation exposure.

## Description

### CROSS-REFERENCE TO RELATED APPLICATIONS

This application claims priority to U.S. Provisional Application No. 60/541,020, which was filed on February 2, 2004, and which is fully incorporated herein by reference.

### BACKGROUND OF THE INVENTION

### 1. Field of the Invention

The present invention relates generally to the field of medical imaging, and, more particularly, to combined intra-rectal optical-magnetic resonance and combined intra-rectal optical-ultrasound device for prostate, cervix and rectum imaging.

### 2. Description of the Related Art

Various diagnostic imaging methods are currently used for the diagnosis of prostate, cervix and rectum cancers. Modern diagnostic imaging techniques include magnetic resonance ("MR"), computer tomography ("CT"), ultrasound ("US") and nuclear medicine (e.g., Positron Emission Tomography ("PET"), Single Photon Emission Computed Tomography ("SPECT")). Anatomical imaging modalities, such as MR and CT, provide adequate anatomical delineation (e.g., bone and soft tissue), but typically do not provide information about specific molecules (e.g., malignant vs. benign tissue) associated with the disease.

On the other hand, optical imaging technologies, such as near-infrared fluorescence ("NIRF"), are promising, emerging modalities that deliver information about specific molecules associated with disease. While the anatomical imaging modalities (e.g., MR, CT) image morphological changes as a result of disease, optical imaging modalities (e.g., NIRF) image and quantify the molecular changes resulting from the disease. For example, molecular changes in cancer may occur up to six years before a mass becomes visible. Therefore, detection of molecular signatures by optical imaging technologies may permit diagnosis at a much earlier stage of the disease, leading to earlier treatment and an increased prognosis. However, optical imaging technologies typically do not deliver sufficient anatomical delineation.

### SUMMARY OF THE INVENTION

In a first aspect of the present invention, an apparatus for providing optical and anatomical diagnostic imaging is provided. The apparatus includes an anatomical imaging unit for inserting into a body cavity, wherein the anatomical imaging unit acquires anatomical images of the body cavity; and an optical imaging unit substantially enclosed within a substantially translucent portion of the anatomical imaging unit, wherein the optical imaging unit detects fluorescence in the body cavity.

In a second aspect of the present invention, an apparatus for providing optical and anatomical diagnostic imaging is provided. The apparatus includes a substantially translucent sheath encompassing a substantially translucent magnetic resonance coil; a head operatively connected to one end of the sheath; and an optical imaging unit inserted through the other end of the sheath, wherein the sheath and the magnetic resonance coil substantially enclosing the optical imaging unit.

In a third aspect of the present invention, apparatus for providing optical and anatomical diagnostic imaging is provided. The apparatus includes a substantially translucent sheath; an ultrasound probe operatively connected to one end of the sheath; and an optical imaging unit inserted through the other end of the sheath, wherein the sheath substantially encloses the optical imaging unit.

In a fourth aspect of the present invention, a method is provided. The method includes inserting a combined optical-anatomical device into a body cavity; and receiving a combined image from the combined optical-anatomical device, wherein the combined image displays anatomical imaging-based information and optical imaging-based information.

### BRIEF DESCRIPTION OF THE DRAWINGS

The invention may be understood by reference to the following description taken in conjunction with the accompanying drawings, in which like reference numerals identify like elements, and in which:
Figure 1 depicts an combined optical-MR device, in accordance with one exemplary embodiment of the present invention;
Figure 2 depicts a combined optical-US device, in accordance with one exemplary embodiment of the present invention;
Figure 3 depicts a handheld component for the combined optical-MR device of Figure 1 and the combined optical-US device of Figure 2.

### DETAILED DESCRIPTION OF PREFERRED EMBODIMENTS

Illustrative embodiments of the invention are described below. In the interest of clarity, not all features of an actual implementation are described in this specification. It will of course be appreciated that in the development of any such actual embodiment, numerous implementation-specific decisions must be made to achieve the developers' specific goals, such as compliance with system-related and business-related constraints, which will vary from one implementation to another. Moreover, it will be appreciated that such a development effort might be complex and time-consuming, but would nevertheless be a routine undertaking for those of ordinary skill in the art having the benefit of this disclosure.

While the invention is susceptible to various modifications and alternative forms, specific embodiments thereof have been shown by way of example in the drawings and are herein described in detail. It should be understood, however, that the description herein of specific embodiments is not intended to limit the invention to the particular forms disclosed, but on the contrary, the intention is to cover all modifications, equivalents, and alternatives falling within the spirit and scope of the invention as defined by the appended claims. It is to be understood that the systems and methods described herein may be implemented in various forms of hardware, software, firmware, special purpose processors, or a combination thereof.

We present, in exemplary embodiments of the present invention, a system combining optical imaging technologies with anatomical imaging technologies (e.g., MR, ultrasound). The system can be used for a variety of applications, including, but not limited to, (1) cancer diagnosis and staging; (2) image guidance; and (3) radiation therapy planning. Image guidance may include guiding a biopsy. For example, a prostatectomy potentially has severe side effects, such as impotence and incontinence. Thus, a histologically-confirmed diagnosis, such as one provided from a biopsy, may prevent unnecessary prostatectomy. Image guidance may also include guiding minimal invasive therapy, such as brachytherapy focused ultrasound. The present invention may be used to plan radiation therapy, for example, by detecting, and thus sparing, healthy tissue from radiation exposure.

We propose a combined optical-anatomical imaging device which is inserted into an accessible body cavity, such as the rectum or the vagina. As used herein, anatomical imaging devices include, but are not limited to, MR-based and ultrasound-based modalities. Optical compounds are integrated into the MR coil and/or the ultrasound head for facilitating the detection of fluorescence, preferably near-infrared fluorescence, thereby enriching the anatomical images obtained from the anatomical imaging devices. The detected fluorescence may result from auto-fluorescence from the body cavity (e.g., intrinsic molecules such as NADP, ATP) or an applied fluorescence probe (i.e., a contrast agent such as ALA or indocyane green) specific to the cancer type under investigation. The detected fluorescence may indicate diseased tissue.

The optical-anatomical imaging device described herein may also be used for detecting targeted and activateable probes (e.g., smart probes). Targeted and activateable probes are further described in Weissleder R., Scaling down imaging: Molecular Mapping of Cancer in Mice. *Nature Rev. Cancer* 2002; 2:11-18, which is fully incorporated herein by reference.

The combined optical-anatomical device is inserted into a body cavity and a combined image displaying anatomical imaging-based information and optical imaging-based information is received.. If a contrast agent is used, the contrast agent may be used prior to the step of inserting. The combined image may be used for any of a variety of practical applications, as contemplated by those skilled in the art, such as neoplasia screening, diagnostics, and therapy monitoring.

### Optical-MR imaging device

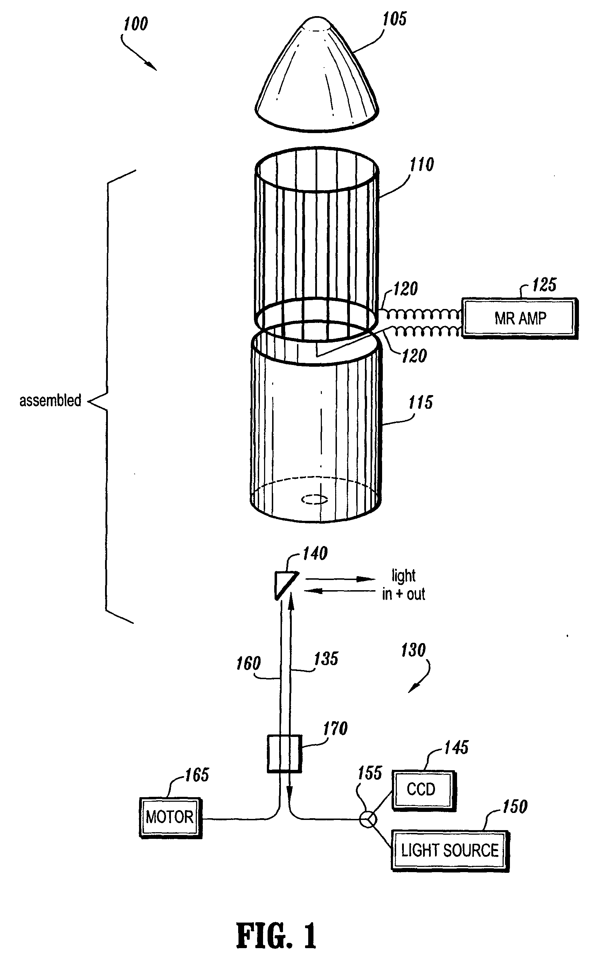

Referring now to Figure 1, an exploded view of an optical-MR device 100 with combined MR imaging and optical imaging functionality is shown, in accordance with one exemplary embodiment of the present invention. The optical-MR device 100 includes a latex head 105, an MR coil 110 and a sheath 115. The latex head 105 is only exemplary. It should be appreciated that any of a variety of soft materials may be used for easy and comfortable insertion into the body cavity. The sheath 115 comprises a rigid housing, preferably transluminescent and filled with a coupling liquid (not shown). The coupling liquid preferably has the same optical index as the surrounding material to prevent bending of illumination light beams (described in greater detail below). The optical-MR device 100 includes two MR wires 120 operatively connected to an amplifier 125. It should be appreciated that, although not shown for the sake of simplicity, the amplifier 125 may be a component of a magnetic resonance tomography ("MRT") unit, as known to those skilled in the art. Further, any number of MR wires may be used as contemplated by those skilled in the art. As described in greater detail below, the optical-MR device 100 further includes an optical component 130. The MR coil 110 is preferably constructed to be translucent (e.g., the spacing between the wires may be made out of a translucent plastic, the coil wires are constructed to be stable without a matrix/support).

In an alternate embodiment, the sheath 115 may be replaced with a light transparent balloon (not shown). The balloon may comprise a foldable investigation head with a liquid pump that fuels rotation. The sheath 115 and the balloon encompass the assembly of MR coil 110 and the optical component 130. The sheath 115 and the balloon may fix the optical-MR device 100 to the body cavity. For example, when the optical-MR device 100 is inserted in the body cavity, the balloon may be inflated. The balloon is preferably inflated with a coupling liquid, but, in an alternate embodiment, may be inflated with air. If a transparent balloon is used, the MR coil 110 (e.g., RF coil, bird cage) is preferably a flexible coil, such as the *MRInnervu*^{*®*} commercially distributed by MEDRAD® Incorporated.

The sheath 115 or the balloon should be positioned such that it covers the terminal detector/illuminator head (e.g., rotating mirror 140, optical head, DMA). Furthermore, the sheath 115 and the balloon may be any of a variety of shapes (e.g. toric) as contemplated by those skilled in the art. The sheath 115 and the balloon are preferably shaped such that the optical-MR device 110 can easily penetrate the particular body cavity being examined. Although only one balloon is described here, it should be appreciated that more than one (e.g., two) balloons may be used, as contemplated by those skilled in the art.

The optical component 130 includes a light wire 135. The light wire 135 may be a single fiber or a fiber bundle. The light wire 135 is inserted through the center of the MR coil 110 and the terminal detector/illuminator head. The light wire 135 is parallel to the MR wires 120. The position of the MR wires 120 are shown in Figure 1 in a manner conducive for easy viewing. However, it should be appreciated that the MR wires 120 may be alternately positioned, as contemplated by those skilled in the art.

On one end of the light wire 135 is a rotating mirror 140, which projects an illumination light circularly on the body cavity (e.g., rectum epithelia) and captures fluorescent light. On the other end of the light wire 135 is an external light source 145 and a light detector 150 connected via the beam splitter 155. Alternatively, the light source 145 and/or the light detector 150 may be integrated within the optical-MR device 100. Although not so limited, the light detector 150 may be a charge-coupled device ("CCD") camera, as shown in Figure 1, or a complimentary metal oxide semiconductor ("CMOS"). The rotating mirror 140 is also connected to one end of a cardan shaft 160. On the other end of the cardan shaft 160 is a motor 165 outside of a magnetic field emanating, for example, from the MRT unit.

As the optical component 130 moves along the MR axis, the rotating mirror 135 captures the fluorescent light over a substantial portion of the body cavity. The optical component 130 may also rotate independently for covering the area around the MR coil. Such rotation may also be used to avoid shadows when the optical-MR device 100 is in a fixed position. In another embodiment, the rotating mirror 135 may be replaced with an optical head (not shown). The optical head may include a circular diffuser or a circular assembly of discrete microscopic lenses. In yet another embodiment, the rotating mirror 135 may be replaced with a digital mirror array ("DMA") or a fish eye, as contemplated by those skilled in the art. Other embodiments may be contemplated by those skilled in the art.

The optical component 130 provides optical imaging for the optical-MR device 100. The optics are preferably produced using nonconductive materials. However, it should be appreciated that conductive material may be used if kept as small and thin as possible for minimizing hyperfine ("HF") coupling.

The optical component 130 illuminates the body cavity and detects fluorescence in the body cavity. The fluorescence may indicate diseased tissue and is distinguished from the illumination. Fluorescent light, which is emitted by an endogenous molecule (which is specific/up-regulated in diseased tissues) or by a contrast agent (which is specifically enriched (e.g., targeted contrast agent) or activated (e.g., smart probe) within the diseased tissue), is at a different wavelength than the illumination light. In general, near infrared light of different wavelength is used for illumination and fluorescence.

Illumination and fluorescent light may be guided by two separate wires (not shown). Alternatively, modulation techniques may be used to distinguish between illumination and fluorescent light. In one embodiment, the optical component 130 may operate in a continuous wave ("CW") mode. Yet alternative designs of the optical component 130 may apply time domain ("TD") measurements, optical tomography ("OCT") or 2-Photone measurements for detecting fluorescence. Tomographic methods may be used for detecting an increase signal-to-noise ratio or increase tissue penetration of the signal to be detected such that fluorescent light/fluorescent markers from deeper parts of the body cavity can be detected.

Illumination may be provided from any of a variety of illumination sources such as broadband light source (e.g., xenon lamp, mercury lamp), light emitting device ("LED") and laser. As previously noted, fluorescence may be provided from any of a variety of fluorescence sources, such as contrast agents and auto-fluorescence.

The intra-rectal measurements obtained from the optical-MR device 100 are preferably combined with an external phased array coils (not shown) for increasing signal-to-noise and increasing the area of the body cavity being examined. The external phased array coils are a typical component of a standard MRT unit. In one embodiment, the external phased array coils may be body arrays, providing ventral and dorsal receiver channels. The body arrays expand the viewable area in the body cavity, which provides information of lymph nodes for, for example, staging.

The optical-MR device 100 may also be combined with monocrystaline iron oxide nanocompounds ("MION"), which is an MR contrast agent. The MION may be dually labeled with a fluorescence dye, which is an optical contrast agent. Further, the MR data set (i.e., the image obtained from the MRT unit using the present invention) may be used for segmentation and subsequence modeling of NIRF absorption and auto-florescence for increasing the NIRF image quality.

### Optical-US imaging device

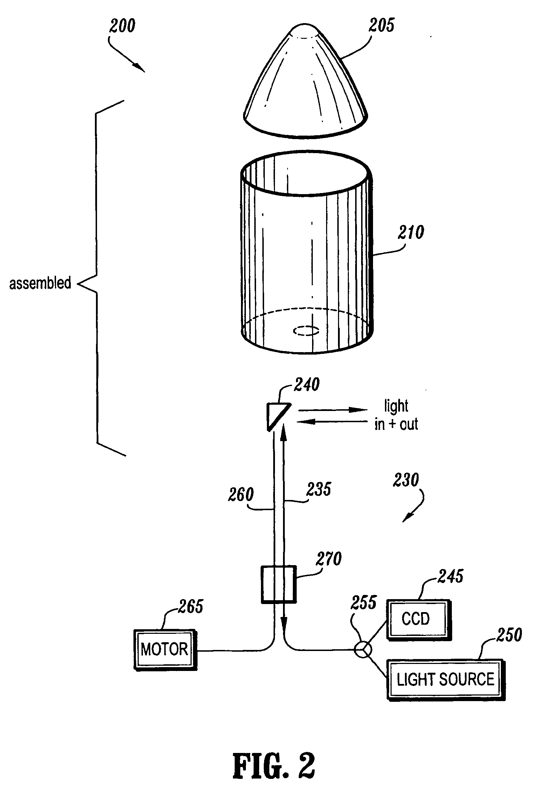

Referring now to Figure 2, an exploded view of an optical-US device 200 with combined ultrasound imaging and optical imaging functionality is shown, in accordance with one exemplary embodiment of the present invention. The optical-US device 200 includes an US probe 205 and a sheath 210. The US probe 205 may be a trans-rectal ultrasound transducer. The sheath 210 is preferably transluminescent and filled with a coupling liquid. The sheath 210 comprises a rigid housing, preferably transluminescent and filled with a coupling liquid (not shown). The coupling liquid preferably has the same optical index as the surrounding material to prevent bending of illumination light beams. The coupling liquid can be used for acoustic coupling (of US) as well. This may be the same liquid or a liquid with a different composition. The optical-US device 200 includes a US wire (not shown) for connecting the US probe 205 to an ultrasound unit (not shown). The optical-US device 200 further includes the optical component 230, as described in greater detail above.

In an alternate embodiment, the sheath 210 may be replaced with a light transparent balloon (not shown). The sheath 210 and the balloon encompass the assembly of the optical component 230. The sheath 210 and the balloon may fix the optical-US device 200 to the body cavity. For example, when the optical-US device 200 is inserted in the body cavity, the balloon may be inflated. The balloon is preferably inflated with a coupling liquid, but, in an alternate embodiment, may be inflated with air.

The sheath 210 or the balloon should be positioned such that it covers the terminal detector/illuminator head (e.g., rotating mirror 240, optical head, DMA). Furthermore, the sheath 210 and the balloon may be any of a variety of shapes (e.g. toric) as contemplated by those skilled in the art. The sheath 210 and the balloon are preferably shaped such that the optical-US device 100 can easily penetrate the particular body cavity being examined. Although only one balloon is described here, it should be appreciated that more than one (e.g., two) balloons may be used, as contemplated by those skilled in the art.

The optical component 230 includes a light wire 235. The light wire 235 may be a single fiber or a fiber bundle. The light wire 235 is inserted through the center of the US probe 205 and the terminal detector/illuminator head. The light wire 235 is parallel to the US wires.

On one end of the light wire 235 is a rotating mirror 240, which projects an illumination light circularly on the body cavity (e.g., rectum epithelia) and captures fluorescent light. On the other end of the light wire 235 is an external light source 245 and a light detector 250 connected via the beam splitter 255. Alternatively, the light source 245 and/or the light detector 250 may be integrated within the optical-US device 200.

Although not so limited, the light detector 250 may be a charge-coupled device ("CCD") camera, as shown in Figure 2, or a complimentary metal oxide semiconductor ("CMOS"). The rotating mirror 240 is also connected to one end of a cardan shaft 260. On the other end of the cardan shaft 260 is a motor 265 outside of a magnetic field emanating, for example, from the ultrasound unit.

As the optical component 230 moves along the MR axis, the rotating mirror 235 captures the fluorescent light over a substantial portion of the body cavity. The optical component 230 may also rotate independently for covering the body cavity area around the optical-US device 200. Such rotation may also be used to avoid shadows when the optical-US device 200 is in a fixed position. In another embodiment, the rotating mirror 235 may be replaced with an optical head (not shown). The optical head may include a circular diffuser or a circular assembly of discrete microscopic lenses. In yet another embodiment, the rotating mirror 235 may be replaced with a digital mirror array ("DMA") or a fish eye, as contemplated by those skilled in the art. Other embodiments may be contemplated by those skilled in the art.

The optical component 230 provides optical imaging for the optical-US device 200. The optics are preferably produced using nonconductive materials. However, it should be appreciated that conductive material may be used if kept as small and thin as possible for minimizing hyperfine ("HF") coupling.

The optical component 230 illuminates the body cavity and detects fluorescence in the body cavity. The fluorescence may indicate diseased tissue. Illumination and fluorescent light may be guided by two separate wires (not shown). Alternatively, modulation techniques may be used to distinguish between illumination and fluorescent light. In one embodiment, the optical component 130 may operate in a continuous wave ("CW") mode. Yet alternative designs of the optical component 130 may apply time domain ("TD") measurements, optical tomography ("OCT") or 2-Photone measurements for detecting fluorescence. Tomographic methods may be used for detecting an increase signal-to-noise ratio or increase tissue penetration of the signal to be detected such that fluorescent light/fluorescent markers from deeper parts of the body cavity can be detected.

Illumination may be provided from any of a variety of illumination sources such as broadband light source (e.g., xenon lamp, mercury lamp), light emitting device ("LED") and laser. As previously noted, fluorescence may be provided from any of a variety of fluorescence sources, such as contrast agents and auto-fluorescence.

The intra-rectal measurements obtained from the optical-US device 200 are preferably combined with an external phased arrays (not shown) for increasing signal-to-noise and increasing the area of the body cavity being examined. The external phased array is a typical component of a standard US unit. In one embodiment, the external phased arrays may be body arrays, providing ventral and dorsal receiver channels. The body arrays expand the viewable area in the body cavity, which provides information of lymph nodes for, for example, staging.

The optical-US device 200 may also be combined with monocrystaline iron oxide nanocompounds ("MION"), which is an MR contrast agent. The MION may be dually labeled with a fluorescence dye, which is an optical contrast agent. Further, the MR data set (i.e., the image obtained from the MRT unit using the present invention) may be used for segmentation and subsequence modeling of NIRF absorption and auto-florescence for increasing the NIRF image quality.

### Handheld component for optical-MR and optical-US devices

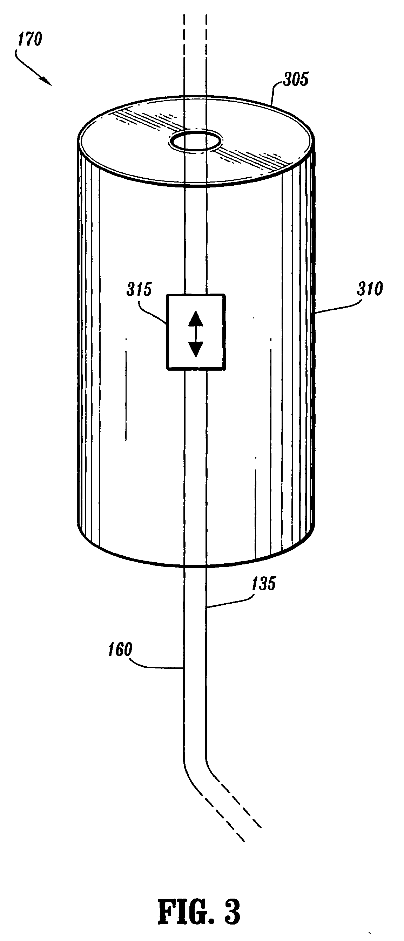

Referring again to Figures 1 and 2, a handheld component 170, 270 is shown encompassing a portion of the optical component 130, 230. It should be appreciated that the handheld component 170, 270 may be positioned several meters from the motor 165, light source 145, 245, and light detector 150, 250. Referring now to Figure 3, the handheld component 170 of Figure 1 is shown, in accordance with one exemplary embodiment of the present invention. Although only the handheld component 170 of Figure 1 is shown in Figure 3, it should be appreciated that the handheld component 270 of Figure 2 operates substantially the same as the handheld component 170 of Figure 1. The handheld component 170 includes an end plate 305, an outer sheath 310 enclosing the light wire 135 and the cardan shaft 160, and a linear motor 315. The outer sheath 310 may be formed as such that a human hand can easily and comfortably grab the outer sheath 310. By gripping the outer sheath 310, a user may maneuver the entire optical-MR 100 or optical-US device 200. The linear motor 315 allows a user to physically maneuver the optical component 130 within a body cavity.

The optical-MR device 100 is typically used in combination with an MRI, preferentially with an open MRI for better access to the patient. If a standard MRI is used (not an open MRI but an MRI with a circular bore), there should be a mechanism for operating the movement of the light wire 135 remotely, since the user may not have access to the handheld component 170. Other uses for a remote control may be contemplated by those skilled in the art.

The movement of the light wire 135 should be reproducible such that an alignment of MR or US with optical imaging technologies, as described herein, is possible. This is especially important for image fusion of MR or US with optical imaging technologies.

The particular embodiments disclosed above are illustrative only, as the invention may be modified and practiced in different but equivalent manners apparent to those skilled in the art having the benefit of the teachings herein. Furthermore, no limitations are intended to the details of construction or design herein shown, other than as described in the claims below. It is therefore evident that the particular embodiments disclosed above may be altered or modified and all such variations are considered within the scope and spirit of the invention. Accordingly, the protection sought herein is as set forth in the claims below.

## Claims

1. An apparatus for providing optical and anatomical diagnostic imaging, comprising:
an anatomical imaging unit for inserting into a body cavity, wherein the anatomical imaging unit acquires anatomical images of the body cavity; and
an optical imaging unit substantially enclosed within a substantially translucent portion of the anatomical imaging unit, wherein the optical imaging unit detects fluorescence in the body cavity.

2. The apparatus of claim 1, wherein the anatomical imaging unit comprises one of a magnetic resonance imaging component for acquiring magnetic resonance images of the body cavity and an ultrasound component for acquiring ultrasound images of the body cavity.

3. The apparatus of claim 1, wherein the optical imaging unit moves along the length of the anatomical imaging unit.

4. The apparatus of claim 1, wherein the optical imaging unit rotates within the anatomical imaging unit.

5. The apparatus of claim 1, wherein the optical imaging unit comprises:
a light wire operatively connected to a terminal detector/illuminator head and a beam splitter, the beam splitter operatively connected to a light source and a light detector; and
a cardan shaft operatively connected to the terminal detector/illuminator head and a motor.

6. The apparatus of claim 5, wherein the terminal detector/illuminator head comprises one of a rotating mirror, an optical head, a digital mirror array, and a fish eye.

7. The apparatus of claim 6, wherein the optical head comprises one of a circular diffuser and a circular assembly of discrete microscopic lenses

8. The apparatus of claim 5, wherein the light source comprises one of a broadband light source, a light emitting device, and a laser.

9. The apparatus of claim 5, wherein the light detector comprises one of charge-coupled device camera or a complimentary metal oxide semiconductor.

10. The apparatus of claim 5, further comprising a handheld component encompassing the light wire and the cardan shaft, the handheld component comprising a linear motor for linearly controlling the optical imaging unit.

11. The apparatus of claim 10, further comprising a remote control for remotely controlling the handheld component.

12. The apparatus of claim 5, wherein movement of the optical imaging unit is reproducible.

13. The apparatus of claim 1, wherein the anatomical imaging unit comprises a magnetic resonance imaging component and an ultrasound imaging component.

14. An apparatus for providing optical and anatomical diagnostic imaging, comprising:
a substantially translucent sheath encompassing a substantially translucent magnetic resonance coil;
a head operatively connected to one end of the sheath; and
an optical imaging unit inserted through the other end of the sheath, wherein the sheath and the magnetic resonance coil substantially enclosing the optical imaging unit.

15. The apparatus of claim 14, wherein the head comprises a latex head.

16. The apparatus of claim 14, wherein the sheath comprises one of a translucent rigid housing and a translucent balloon.

17. The apparatus of claim 14, wherein the sheath is filled with a coupling liquid.

18. An apparatus for providing optical and anatomical diagnostic imaging, comprising:
a substantially translucent sheath;
an ultrasound probe operatively connected to one end of the sheath; and
an optical imaging unit inserted through the other end of the sheath, wherein the sheath substantially encloses the optical imaging unit.

19. The apparatus of claim 18, wherein the translucent sheath comprises one of a translucent rigid housing and a translucent balloon.

20. The apparatus of claim 18, wherein the translucent sheath is filled with a coupling liquid.

21. The apparatus of claim 18, wherein the ultrasound probe comprises a trans-rectal ultrasound transducer.

22. A method, comprising:
inserting a combined optical-anatomical device into a body cavity; and
receiving a combined image from the combined optical-anatomical device, wherein the combined image displays anatomical imaging-based information and optical imaging-based information.

23. The method of claim 22, wherein the step of inserting a combined optical-anatomical device into a body cavity comprises one of inserting a combined optical-MR device and inserting a combined optical-US device.

24. The method of claim 22, further comprising injecting a contrast agent prior to the step of inserting.

25. The method of claim 22, further comprising analyzing the combined image for one of neoplasia screening, diagnostics, and therapy monitoring.
